# EUROPÄISCHE PATENTSCHRIFT

(11) **EP 0 602 377 B1**
(45) Veröffentlichungstag und Bekanntmachung des Hinweises auf die Patenterteilung: **09.10.1996**
(21) Anmeldenummer: 93117980.8
(22) Anmeldetag: 05.11.1993
(51) Int. Cl.: C07C 311/53, C08F 22/38, G03F 7/038

(54) **Polymere mit N,N-disubstituierten Sulfonamid-Seitengruppen und deren Verwendung in strahlungsempfindlichen Gemischen**
Polymers containing N,N-disubstituted sulfonamide side groups and their use in radiation-sensitive compositions
Polymères contenant des sulfonamides N,N-disubstitués comme groupes latéraux et leur utilisation dans des compositions sensibles aux radiations

(30) Priorität: 14.12.1992 DE 4242050
(43) Veröffentlichungstag der Anmeldung: 22.06.1994
(73) Patentinhaber: HOECHST AKTIENGESELLSCHAFT, 65926 Frankfurt am Main (DE)
(72) Erfinder: Eichhorn, Mathias, Dr., D-65527 Niedernhausen (DE); Buhr, Gerhard, Dr., D-61462 Königstein (DE)

(56) Entgegenhaltungen:
- EP-A- 0 102 450
- EP-A- 0 421 388

## Beschreibung

Polymere mit N,N-disubstituierten Sulfonamid-Seitengruppen und deren Verwendung

Die Erfindung betrifft Monomere mit N,N-disubstituierten Sulfonamidgruppen, Polymere, die N,N-disubstituierte Sulfonamid-Seitengruppen aufweisen, und ein strahlungsempfindliches Gemisch, das
a) eine unter der Einwirkung aktinischer Strahlung Säure bildende Verbindung,
b) eine säurespaltbare Verbindung, deren Spaltprodukte in einem wäßrig-alkalischen Entwickler eine höhere Löslichkeit zeigen als die Ausgangsverbindung, und
c) ein in Wasser unlösliches, in wäßrig-alkalischen Lösungen dagegen lösliches, zumindest quellbares polymeres Bindemittel
enthält. Es eignet sich besonders für ein Aufzeichnungsmaterial aus einem Träger und einer strahlungsempfindlichen Schicht zur Herstellung von Offsetdruckplatten und Photoresists.

Positiv arbeitende strahlungsempfindliche Aufzeichnungsschichten, d. h. Schichten, deren Löslichkeit in den bestrahlten Bereichen größer ist als in den unbestrahlten, sind bekannt. Als lichtempfindliche Komponente in solchen Schichten haben sich insbesondere ortho-Naphthochinondiazide durchgesetzt. Die Lichtempfindlichkeit dieser Schichten ist jedoch meist unbefriedigend.

Die sogenannten "chemisch verstärkten" Gemische zeigen demgegenüber eine höhere Lichtempfindlichkeit, da die Quantenausbeute größer als 1 ist. Positiv arbeitende, "chemisch verstärkte" Gemische enthalten in der Regel eine säurebildende und eine säurespaltbare Komponente, deren Spaltprodukte in wäßrig-alkalischen Entwicklern eine höhere Löslichkeit als die Ursprungsverbindung besitzen.

Als säurespaltbare Verbindungen wurden bisher monomere und polymere Acetale und O,N-Acetale, die als Hydroxyl- oder Aminkomponente aromatische Verbindungen enthalten (US-A 3 779 778), sowie Orthoester und Amidacetale (DE-B 26 10 842 = US-A 4 101 323) verwendet. Strahlungsempfindliche positiv arbeitende Gemische erhält man auch unter Verwendung von polymeren Orthoestern (EP-B 0 022 571 = US-A 4 311 782), polymeren aliphatischen Acetalen (DE-A 27 18 254 = US-A 4 247 611), Enolethern (EP-B 0 006 627 = US-A 4 248 957) sowie N-Acyliminocarbonaten (EP-B 0 006 626 US-A 4 250 247). Derartige Gemische benötigen zur Auslösung der Spaltreaktion neben der photochemisch erzeugten Säure auch Wasser, was zu Problemen in der praktischen Verwendbarkeit führt. Außerdem sind viele der genannten Verbindungen nur schwer zugänglich.

Ein positiv arbeitendes strahlungsempfindliches Gemisch, das eine Verbindung, die bei Bestrahlung Säure hervorbringt, und ein Polymer mit seitenständigen, säurelabilen tert.-Butoxycarbonyl- oder tert.-Butoxycarbonyloxy-Gruppen, enthält ist in der EP-A 0 102 450 und in der EP-A 0 366 590 beschrieben. Ein ähnliches Gemisch, das jedoch anstelle der genannten Polymere niedermolekulare Verbindungen mit säurelabilen Gruppen enthält, ist in der EP-A 0 249 139 offenbart. Noch änhliche strahlungsempfindliche Gemische, die zwei Copolymere enthalten, sind aus EP-A-0 421 388 bekannt. Als säurelabile Gruppen in den niedermolekularen Verbindungen, die allgemein ein Molekulargewicht von weniger als 1.000 haben, sind insbesondere tert.-Butoxy-, tert.-Butoxycarbonyl-, tert.-Butoxycarbonyloxy-, 1-Methyl-1-phenyl-ethoxycarbonyl- und Trimethylsilanyloxy-Gruppen offenbart.

Solche Systeme benötigen zwar kein Wasser für die Auslösung der Spaltreaktion, sind aber auch nicht frei von Nachteilen: Sie zeigen z. B. einen relativ hohen "Dunkelabtrag", d. h. die Löslichkeit der strahlungsempfindlichen Schicht in einem Entwickler ist auch in den unbelichteten Bereichen relativ hoch, was zu einer schlechten Differenzierung zwischen belichteten und unbelichteten Bereichen führt.

Aufgabe der Erfindung war es, einfach und kostengünstig herstellbare, säurespaltbare Verbindungen zu entwickeln, die sich besonders gut für ein positiv-arbeitendes, "chemisch verstärktes" Gemisch eignen, das eine hohe Empfindlichkeit gegenüber aktinischer Strahlung - insbesondere der von Excimer-Lasern und Quecksilberhochdruckdampflampen emittierten Strahlung - aufweist.

Erfindungsgemäß wird diese Aufgabe gelöst durch die Bereitstellung von Monomeren mit N,N-disubstituierten Sulfonamidgruppen der Formeln -R³-N(CO-OR²)-SO₂-R¹ (I) oder -R³-SO₂-N(CO-OR²)-R¹ (II) sowie von Polymeren mit mindestens 5 mol-% an Einheiten mit Seitengruppen der Formel(n) I und/oder II worin
- R¹: ein (C₁-C₂₀)Alkyl-, (C₃-C₁₀)Cycloalkyl-, (C₆-C₁₄)Aryl- oder (C₇-C₂₀)Aralkylrest, wobei in den Alkyl enthaltenden Resten einzelne Methylengruppen durch Heteroatome ersetzt sein können, und
- R²: ein (C₃-C₁₁)Alkyl-, (C₃-C₁₁)Alkenyl- oder (C₇-C₁₁)Aralkylrest,
- R³: ein unsubstituierter oder substituierter (C₁-C₁₂)Alkylen-, (C₃-C₁₂)Cycloalkylen-, (C₆-C₁₅)Arylen- oder (C₈-C₂₀)Arylendialkylrest ist.

R² ist bevorzugt ein (C₃-C₆)Alkylrest, besonders bevorzugt ein Isopropyl, sec.-Butyl- oder tert.-Butylrest.

Wie die Beispiele zeigen, enthalten die Polymere bevorzugt 10 bis 90 mol-%, besonders bevorzugt 20 bis 80 mol-% an Einheiten mit Gruppen der Formel I oder II. Copolymere sind somit allgemein gegenüber Homopolymeren bevorzugt. Die Polymere haben allgemein ein Molekulargewicht im Bereich von 2.000 bis 100.000, bevorzugt von 5.000 bis 50.000.

Als Comonomere eignen sich im Prinzip alle polymerisierbaren Verbindungen ohne säurespaltbare Gruppen. "Nichtsäurespaltbar" bedeutet in diesem Zusammenhang, daß die Gruppen von der in dem erfindungsgemäßen Gemisch erzeugten Säure nicht gespalten werden. Solche Comonomere sind beispielsweise Acrylsäure, Methacrylsäure, (C₁-C₁₀)Alkyl-acrylate und -methacrylate, (C₆-C₁₀)Aryl-acrylate und -methacrylate (besonders Phenylacrylat, Phenylmethacrylat und Brenzkatechin-mono-methacrylat), Acrylamid, Methacrylamid, gegebenenfalls substituiertes N-(C₁-C₁₀)Alkyl-acrylamid und -methacrylamid (besonders N-(2-Hydroxy-ethyl)-acrylamid und -methacrylamid), N,N-Di-(C₁-C₁₀)alkyl-acrylamid, N-Phenyl-acrylamid und -methacrylamid, N,N-Diphenyl-acrylamid und -methacrylamid, N-(C₁-C₁₀)Alkyl-N-(C₆-C₁₀)Aryl-acrylamide (besonders N-Phenyl-N-methyl-acrylamid und -methacrylamid), N-Phthalimidomethyl-methacrylamid, Allylverbindungen, wie Allylester und Allyloxy-ethanol, Vinylverbindungen, wie Vinylether und Vinylester, Vinylaromaten, wie Styrol, Alkylstyrol, Alkoxystyrol, Hydroxystyrol, Alkyl-hydroxystyrol, Acetoxystyrol, α-Methyl-styrol, α-Methyl-hydroxy-styrol, schließlich auch Acrylnitril und Methacrylnitril. Weniger bevorzugt, doch unter Umständen zweckmäßig, ist die Verwendung von Comonomeren mit säurespaltbaren Gruppen.

Bevorzugte Monomere mit N,N-disubstituierten Sulfonamidgruppen der Formel I sind R¹-SO₂-N(CO-OR²)-R³-O-CO-CR⁴=CH₂, bevorzugte Monomere mit Gruppen der Formel II sind R¹-N(CO-OR²)-SO₂-R³-O-CO-CR⁴=CH₂, worin R⁴ für ein Wasserstoffatom oder eine Methylgruppe steht. Die bevorzugten Monomere sind somit Acrylate oder Methacrylate.

Die Monomere mit N,N-disubstituierten Sulfonamidgruppen können aus den entsprechenden Verbindungen mit N-monosubstituierten Sulfonamidgruppen hergestellt werden, indem man diese in Gegenwart einer katalytischen Menge einer organischen Base, wie 4-Dimethylamino-pyridin, mit aktivierten Kohlensäureestern, die die Gruppierung -OR² als Alkoholkomponente enthalten, umsetzt.

N-Monosubstituierte Sulfonamide sind nach Verfahren, die dem Fachmann bekannt sind, aus Sulfonsäuren und primären Aminen herstellbar. Für die Herstellung der Monomere mit Gruppen der Formel II werden allgemein Monoamine verwendet. Bevorzugte Monoamine sind geradkettige oder verzweigte Alkylamine mit 1 bis 12, besonders bevorzugt 1 bis 6, Kohlenstoffatomen, wie Methylamin, Ethylamin, Propylamin, Isopropylamin, Butylamin, sec.-Butylamin, Isobutylamin, tert.-Butylamin, Pentylamin, 1-Methyl-butylamin, 2-Methyl-butylamin und Hexylamin. Bevorzugt sind daneben auch Cycloalkylamine mit 3 bis 12, besonders bevorzugt 5 bis 8, Kohlenstoffatomen, wie Cyclopentylamin, Cyclohexylamin, Cycloheptylamin und Cyclooctylamin. Von den aromatischen Monoaminen sind solche mit 6 bis 15 Kohlenstoffatomen bevorzugt, deren aromatischer Teil substituiert sein kann, besonders mit Halogenatomen, Alkyl- oder Alkoxygruppen. Beispiele für bevorzugte aromatische Amine sind Anilin, 4-Methyl-anilin, 4-Ethyl-anilin, 4-Methoxy-anilin, 3-Methoxy-anilin, 4-Ethoxy-anilin, 4-Phenoxy-anilin, Naphthylamin, Biphenylamin, 1- und 2-Amino-anthracen und 9-Amino-phenanthren. Von den Aralkylaminen sind solche mit 7 bis 20 Kohlenstoffatomen bevorzugt. Diese können in der gleichen Weise wie die aromatischen Amine substituiert sein. Beispiele hierfür sind Benzylamin, 4-Methoxy-benzylamin, 2,2- und 3,3-Diphenyl-propylamin.

Für die Herstellung der Monomere mit Gruppen der Formel I werden primäre Amine mit einer polymerisierbaren olefinisch ungesättigten Gruppe oder mit einer funktionellen Gruppe, die die Bindung zu einer solchen polymerisierbaren Gruppe herstellt, eingesetzt. Für die Herstellung der oben erwähnten Acrylate und Methacrylate sind Hydroxyamine, wie Ethanolamin, besonders geeignet. Durch Umsetzung der Hydroxygruppe mit beispielsweise Methacrylsäureanhydrid kann dann die polymerisierbare Doppelbindung eingeführt werden.

Für die zur Herstellung der Monomeren verwendeten Sulfonsäuren gilt das Entsprechende wie für die Amine. Zur Herstellung der Monomeren mit Gruppen der Formel I bevorzugte Sulfonsäuren sind Methansulfonsäure, Trifluormethansulfonsäure, Ethansulfonsäure, Propansulfonsäure, Perfluorpropansulfonsäure, Perfluorbutansulfonsäure, Hexansulfonsäure, Perfluoroctansulfonsäure, Benzolsulfonsäure, Pentafluorbenzolsulfonsäure, para-Toluolsulfonsäure und Naphthalinsulfonsäure. Zur Herstellung der Monomere mit Gruppen der Formel II werden demgemäß Sulfonsäuren verwendet, die in der gleichen Weise funktionalisiert sind wie die Amine zur Herstellung der Monomere mit Gruppen der Formel I. Ein Beispiel für eine funktionalisierte Sulfonsäure ist 4-Hydroxy-benzolsulfonsäure.

Die Sulfonsäuren werden allgemein nicht als solche in die Reaktion eingesetzt, sondern in einer reaktionsfähigeren, sogenannten "aktivierten" Form. Hierzu zählen besonders die Sulfonylhalogenide, speziell die Sulfonylchloride.

Als "aktivierte Kohlensäureester" werden solche bezeichnet, die eine Acylierung der N-monosubstituierten Sulfonamide mit -CO-OR² bewirken können. Dies sind insbesondere Dialkyldicarbonate (= Pyrokohlensäure-dialkylester). Besonders bevorzugt ist Di-tert.-butyldicarbonat (= O[CO₂-C(CH₃)₃]₂).

Die Umsetzung der N-monosubstituierten Sulfonamide mit den aktivierten Kohlensäureestern erfolgt vorteilhaft in einem Lösemittel, das unter den Reaktionsbedingungen keine irreversiblen Reaktionen mit den übrigen Bestandteilen des Reaktionsgemisches eingeht, in Gegenwart von 0,01 bis 10 mol-%, bevorzugt 0,05 bis 2 mol-%, jeweils bezogen auf die molare Menge an N-monosubstituiertem Sulfonamid, einer organischen Base. Diese Base ist bevorzugt ein tertiäres Amin, z. B. Dialkylaminopyridin. Geeignete Lösemittel sind insbesondere Tetrahydrofuran, Ethylacetat, Diethylether, Butanon (= Methylethylketon). Es hat sich als zweckmäßig erwiesen, das N-monosubstituierte Sulfonamid und die organische Base in gelöster Form vorzulegen und zu diesem Gemisch den aktivierten Kohlensäureester langsam zuzugeben. Die Reaktion wird im allgemeinen bei einer Temperatur von 0 bis 80 °C, bevorzugt 10 bis 50 °C, durchgeführt. Das Reaktionsprodukt kann dann durch Eingießen des Reaktionsgemisches in Wasser, Abfiltrieren des Niederschlags und Trocknen, oder einfach durch Abziehen der flüchtigen Bestandteile unter vermindertem Druck in ausreichender Reinheit isoliert werden. Falls erforderlich, kann durch Umkristallisieren, Umfällen, Destillieren oder durch ein präparatives chromatographisches Verfahren eine weitere Reinigung erfolgen.

Erfindungsgemäß wird weiterhin ein strahlungsempfindliches Gemisch vorgeschlagen, das
a) eine Verbindung, die unter der Einwirkung aktinischer Strahlung Säure bildet, und
b) eine säurespaltbare Verbindung, deren Spaltprodukte in einem wäßrig-alkalischen Entwickler eine höhere Löslichkeit zeigen als die Ausgangsverbindung,
enthält und dadurch gekennzeichnet ist, daß die säurespaltbare Verbindung b) ein Polymer mit mindestens 5 mol-% an Einheiten mit N,N-disubstituierten Sulfonamid-Seitengruppen der allgemeinen Formel I oder II ist.

Als Verbindungen a), die unter der Einwirkung aktinischer Strahlung vorzugsweise starke Säuren bilden, sind u. a. insbesondere Diazonium-, Phosphonium-, Sulfonium- und Iodoniumsalze, Halogenverbindungen, o-Chinondiazidsulfochloride, -ester und -amide sowie Organometall/Organohalogen-Kombinationen geeignet. Die genannten Diazonium-, Phosphonium-, Sulfonium- und Iodonium- verbindungen werden in der Regel eingesetzt in Form ihrer in organischen Lösemitteln löslichen Salze, insbesondere in Form der Sulfonate, speziell Trifluormethansulfonate, der Tetrafluorborate, Hexafluorphosphate, Hexafluorantimonate oder Hexafluorarsenate. Es können aber auch Halogenide, Ester und Amide von 1,2-Naphthochinon-2-diazid-sulfonsäuren verwendet werden. Die Azidität der beim Bestrahlen von o-Naphthochinondiaziden entstehenden Indencarbonsäuren reicht jedoch meist nur knapp für eine ausreichende bildmäßige Differenzierung aus. Bevorzugt wird daher aus dieser Gruppe das 1,2-Napthochinon-2-diazid-4-sulfonylchlorid, bei dessen Bestrahlung drei Säurefunktionen gebildet werden, so daß ein relativ großer Verstärkungsfaktor besteht. Schließlich sind als Säurebildner auch organische Halogenverbindungen brauchbar, beispielsweise solche mit mehr als einem Halogenatom an einem Kohlenstoffatom oder an einem aromatischen Ring. Die spektrale Empfindlichkeit dieser halogenhaltigen Verbindungen kann durch an sich bekannte Sensibilisatoren verändert und gesteigert werden. Beispiele für besonders geeignete Säurebildner sind 1,2-Naptho-chinon-2-diazid-4-sulfonylchlorid; 4-Dipropylamino-benzoldiazoniumtetrafluorborat, -hexafluorphosphat und -trifluormethansulfonat; 2,5-Diethoxy-4-p-tolylmer-capto-benzoldiazoniumtetrafluorborat, -hexafluorphosphat und -trifluormethansulfonat; 4-Anilino-benzoldiazoniumsulfat und 4-Diethylamino-benzoldiazoniumtrifluormethansulfonat sowie die in den Beispielen angeführten Verbindungen. Ebenfalls verwendet werden können 4-Methyl-6-trichlormethyl-2-pyron; 4-(3,4,5-Trimethoxy-styryl)-6-trichlormethyl-2-pyron; 4-(4-Methoxy-styryl)-6-(3,3,3-trichlorpropenyl)-2-pyron; 2-Trichlormethylbenzimidazol; 2-Tribrommethylchinolin-4-on; 2,4-Di-methyl-1-tribromacetyl-benzol; 3-Nitro-1-tribromacetylbenzol; 4-Dibromacetylbenzoesäure; 1,4-Bisdibrommethylbenzol; substituierte 4,6-Bis-trichlormethyl-s-triazine wie 2-(6-Methoxy-naphthalin-2-yl)-, 2-(Naphthalin-1-yl)-, 2-(Naphthalin-2-yl)-, 2-[4-(2-Ethoxy-ethyl)-naphthalin-1-yl]-, 2-Benzopyran-3-yl-, 2-Phenanthren-9-yl- und 2-(4-Methoxy-anthracen-1-yl)-4,6-bis-trichlormethyl-s-tri-azin und Tris-dibrommethyl-s-triazin.

Der Anteil der säurebildenden Verbindung(en) a) an dem Gemisch ist je nach Zusammensetzung des Gemisches unterschiedlich. Günstige Ergebnisse werden erhalten mit etwa 0,1 bis 20 Gew.%, bevorzugt 0,2 bis 10% Gew.-%, jeweils bezogen auf das Gesamtgewicht der Feststoffe in dem Gemisch. Besonders für Kopierschichten mit einer Dicke von mehr als 10 µm empfiehlt es sich, relativ wenig Säurebildner zu verwenden.

Der Anteil der säurespaltbaren Polymeren b) beträgt im allgemeinen 30 bis 98 Gew.-%, bevorzugt 50 bis 95 Gew.-%, jeweils bezogen auf das Gesamtgewicht der Feststoffe in dem Gemisch.

Neben den erfindungsgemäßen können noch andere säurespaltbare Verbindungen in dem Gemisch vorhanden sein. Dies sind insbesondere Polymere mit tert.-Butoxycarbonyl-Gruppen. Gemische mit solchen weiteren säurespaltbaren Verbindungen sind allgemein jedoch nicht bevorzugt.

Gegebenenfalls kann das Gemisch daneben noch polymere organische Bindemittel enthalten. Besonders geeignete polymere Bindemittel sind Phenolharze, insbesondere Kresol-Formaldehyd-Novolake (Schmelzbereich 105-120°C nach DIN 53181) und Phenol-Formaldehyd-Novolake (Schmelzbereich 110-120°C nach DIN 53181).

Art und Anteil des Bindemittels richten sich nach dem Anwendungszweck. Bevorzugt ist ein Anteil von 5 bis 70 Gew.-%, besonders bevorzugt von 20 bis 50 Gew.-%, jeweils bezogen auf das Gesamtgewicht der Feststoffe im Gemisch.

Bindemittel, deren Alkalilöslichkeit durch Einwirkung von Säure erhöht wird, sind im erfindungsgemäßen Gemisch ebenfalls verwendbar. Solche Bindemittel können z.B. Polyhydroxystyrole sein, deren phenolische OH-Gruppen mit säurelabilen Gruppierungen versehen sind, die die Alkalilöslichkeit herabsetzen. Die erfindungsgemäßen Verbindungen bewirken eine deutliche Verringerung des Dunkelabtrags, ohne die Lichtempfindlichkeit des Gemisches zu beeinträchtigen.

Andere alkalilösliche Harze, wie Copolymere aus Methacrylsäure und Methylmethacrylat, Vinylacetat und Crotonsäure, sowie Maleinsäureanhydrid und Styrol, sind als Bindemittel ebenfalls geeignet.

Zusätzlich können noch zahlreiche andere Harze mitverwendet werden, bevorzugt Vinylpolymere wie Polyvinylacetate, Polyacrylate, Polyvinylether und Polyvinylpyrrolidone, die wiederum durch Comonomere modifiziert sein können. Der günstigste Anteil an diesen Harzen richtet sich nach den anwendungstechnischen Erfordernissen und dem Einfluß auf die Entwicklungsbedingungen. Er beträgt im allgemeinen nicht mehr als 50 Gew.-%, bezogen auf das Gesamtgewicht der Feststoffe in dem Gemisch.

Um spezielle Erfordernisse, wie Flexibilität, Haftung, oder Glanz zu erfüllen, kann das strahlungsempfindliche Gemisch daneben noch Substanzen wie Polyglykole, Cellulose-Derivate, wie Ethylcellulose, Netzmittel, Farbstoffe und feinteilige Pigmente enthalten. Als Farbstoffe haben sich die Triphenylmethanfarbstoffe, insbesondere in Form ihrer Carbinolbasen, besonders bewährt. Die günstigsten Mengenverhältnisse der Komponenten lassen sich im Einzelfall durch Versuche leicht ermitteln.

Gegenstand der vorliegenden Erfindung ist schließlich auch ein Aufzeichnungsmaterial mit einem Träger und einer strahlungsempfindlichen Schicht aus dem erfindungsgemäßen Gemisch. Das Aufzeichnungsmaterial wird üblicherweise durch Beschichten des Trägers mit einer Lösung des Gemisches hergestellt.

Geeignete Lösemittel für das erfindungsgemäße strahlungsempfindliche Gemisch sind Ketone wie Methylethylketon, chlorierte Kohlenwasserstoffe wie Trichlorethylen und 1,1,1-Trichlorethan, Alkohole wie n-Propanol, Ether wie Tetrahydrofuran, Glykolether wie Ethylenglykolmonoethylether und Ester wie Butylacetat. Es können auch Gemische verwendet werden, die zudem noch für spezielle Zwecke Lösemittel wie Acetonitril, Dioxan oder Dimethylformamid enthalten können. Prinzipiell sind alle Lösemittel verwendbar, die mit den Schichtkomponenten nicht irreversibel reagieren. Sie sind jedoch im Hinblick auf das vorgesehene Beschichtungsverfahren, die Schichtdicke und die Trocknungsvorrichtung auszuwählen. Dünne Schichten bis ca. 5 µm werden für Versuchsmengen vorzugsweise aufgeschleudert. Schichtdicken von mehr als 60 µm sind mit Lösungen bis zu ca. 40 % Feststoffgehalt bei einmaligem Auftrag auf der Schleuder oder mit einer Streichrakel erreichbar. Für beidseitiges Beschichten wird Tauchbeschichtung vorgezogen, wobei schnelles Antrocknen von Vorteil ist, das durch Verwendung niedrigsiedender Lösemittel erreicht wird. Bandförmige Trägermaterialien können durch Aufsprühen der Beschichtungslösung mit Breitschlitzdüsen oder Antragen mit Walzen, Einzelplatten - wie Zink- und Mehrmetallplatten - durch Gießantrag (curtain coating) beschichtet werden.

Im Vergleich zu anderen Positivschichten, besonders denen auf o-Naphthochinondiazidbasis, ist auch die Herstellung dickerer Schichten möglich, da die Lichtempfindlichkeit der erfindungsgemäßen Gemische verhältnismäßig wenig dickenabhängig ist. Belichtung und Verarbeitung von Schichten einer Dicke von 100 µm und darüber sind möglich.

Bevorzugte Träger für Schichten von mehr als 10 µm Dicke sind Kunststoffolien, die dann als temporäre Träger für Transferschichten dienen. Dafür und für Farbfolien werden Polyesterfolien, z. B. aus Polyethylenterephthalat, bevorzugt. Polyolefinfolien wie Polypropylen sind jedoch ebenfalls geeignet. Als Schichtträger für Schichtdicken von weniger als 10 µm werden meist Metalle verwendet. Zur Herstellung von Offsetdruckplatten kann mechanisch oder elektrochemisch aufgerauhtes und gegebenenfalls anodisiertes Aluminium, das evtl. chemisch (z. B. mit Polyvinylphosphonsäure, Silikaten oder Phosphaten) vorbehandelt ist, verwendet werden. Geeignet sind auch Mehrmetallplatten mit Cu/Cr oder Messing/Cr als oberster Schicht. Für Hochdruckplatten können die aus dem erfindungsgemäßen Gemisch hergestellten Schichten auf Zink- oder Magnesiumplatten sowie deren handelsübliche mikrokristalline Legierungen für Einstufenätzverfahren aufgetragen werden, außerdem auf ätzbare Kunststoffe wie Polyoxymethylen. Für Tiefdruck- oder Siebdruckformen eignen sich diese Schichten durch ihre gute Haftung und Ätzfestigkeit auf Kupfer- bzw. Nickeloberflächen. Ebenso lassen sie sich als Photoresists und beim Formteilätzen einsetzen.

Schließlich kann die Beschichtung direkt oder durch trockene Schichtübertragung vom temporären Träger erfolgen auf Leiterplattenmaterialien, die aus Isolierplatten mit ein- oder beidseitiger Kupferauflage bestehen, auf Glas- oder Keramikmaterialien, die ggf. haftvermittelnd vorbehandelt sind, und u. a. auf Siliciumscheiben, auf deren Oberfläche sich ggf. eine Nitrid- oder Oxidschicht befindet. Außerdem können Holz, Textilien und Oberflächen vieler Werkstoffe beschichtet werden, die vorzugsweise durch Projektion bebildert werden und resistent gegen die Einwirkung alkalischer Entwickler sind.

Die Beschichtung kann mit den üblichen Geräten und unter den üblichen Bedingungen getrocknet werden. Sie verträgt Temperaturen um 100 °C, kurzfristig sogar bis zu 120 °C, ohne daß später eine Einbuße an Strahlungsempfindlichkeit beobachtet wird.

Zum Bestrahlen können die üblichen Strahlungsquellen, wie Röhrenlampen, Xenonimpulslampen, metallhalogeniddotierte Quecksilberdampf-Hochdrucklampen und Kohlebogenlampen verwendet werden. Darüber hinaus ist das Bestrahlen in üblichen Projektions- und Vergrößerungsgeräten unter dem Licht der Metallfadenlampen und Kontaktbelichtung mit gewöhnlichen Glühbirnen möglich. Die Bestrahlung kann auch mit dem kohärenten Licht eines Lasers erfolgen. Geeignet sind hier leistungsfähige kurzwellige Laser, beispielsweise Argon-Ionen-Laser, Krypton-Ionen-Laser, Farbstoff-Laser, Helium-Cadmium-Laser sowie Excimer-Laser, die zwischen 193 und 633 nm emittieren. Der Laser wird üblicherweise computergesteuert raster- oder strichartig über die Aufzeichnungsschicht geführt und bestrahlt diese dabei bildmäßig.

Das Bestrahlen mit Elektronenstrahlen ist eine weitere Bebilderungsmöglichkeit. Elektronenstrahlen können das erfindungsgemäße Gemisch wie auch viele andere organische Materialien durchgreifend zersetzen und anschließend vernetzen, so daß ein negatives Bild entsteht, wenn die unbestrahlten Teile durch Lösemittel oder Belichten ohne Vorlage und Entwickeln entfernt werden. Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die Wahl der günstigsten Bedingungen kann durch Versuche leicht ermittelt werden.

Die bildmäßig belichtete oder bestrahlte Schicht kann, gegebenenfalls nach einer thermischen Nachbehandlung, mit praktisch den gleichen Entwicklern wie für handelsübliche Naphthochinondiazidschichten und Kopierlacke entfernt werden, bzw. die neuen Schichten können in ihren Kopierbedingungen vorteilhaft auf die bekannten Hilfsmittel, wie Entwickler und programmierte Sprühentwicklungsgeräte, abgestimmt werden. Die wäßrigen Entwicklerlösungen können z. B. Alkaliphosphate, -silikate oder -hydroxide und ferner Netzmittel sowie kleinere Anteile organischer Lösemittel enthalten. In bestimmten Fällen sind anstelle der wäßrig-alkalischen Entwickler auch organische Lösemittel oder Gemische von organischen Lösemitteln mit Wasser als Entwickler brauchbar. Bevorzugt als Entwickler sind jedoch wäßrig-alkalische Lösungen.

Der günstigste Entwickler kann durch Versuche mit der jeweils verwendeten Schicht ermittelt werden. Bei Bedarf kann die Entwicklung mechanisch unterstützt werden. Zur Erhöhung der Widerstandsfähigkeit beim Druck sowie der Resistenz gegen Auswaschmittel, Korrekturmittel und durch UV-Licht härtbare Druckfarben können die entwickelten Platten kurze Zeit auf erhöhte Temperaturen erwärmt werden.

Im folgenden werden Beispiele für die bevorzugten erfindungsgemäßen Monomeren mit N,N-disubstituierten Sulfonamidgruppen, für damit hergestellte Polymere und für bevorzugte, mit den Polymeren hergestellte, lichtempfindlichen Gemische angegeben, ohne die Erfindung auf diese zu beschränken. Gt steht in den Beispielen für Gewichtsteile.

### Herstellung von Monomeren

### I. N-tert.-Butoxycarbonyl-N-(2-methacryloyloxy-ethyl)-p-toluolsulfonamid

a) 100 g p-Toluolsulfonylchlorid werden langsam im Verlauf von 1,5 h unter Eiskühlung zu 80 ml Ethanolamin gegeben. Anschließend wird das Reaktionsgemisch auf 120 °C erhitzt und dann 3 h lang bei dieser Temperatur gehalten. Nach dem Abkühlen auf 70 °C wird ein Gemisch aus 60 ml konz. HCl und 300 ml Wasser zugegeben. Nach 10 min. Rühren werden 300 ml Methylenchlorid zugegeben, dann wird die organische Phase abgetrennt und mit MgSO₄ getrocknet. Nach dem Abziehen des Lösemittels am Rotationsverdampfers erhält man 111,6 g eines leicht gelblichen Feststoffs [N-(2-Hydroxy-ethyl)-p-toluolsulfonamid]. Das Produkt kann ohne weitere Reinigung in der nächsten Stufe eingesetzt werden.
b) 6,8 ml Methacrylsäure-anhydrid und 6,3 ml Triethylamin werden tropfenweise unter Eiskühlung zu einer Lösung aus 8,9 g N-(2-Hydroxy-ethyl)-p-toluolsulfonamid in 50 ml Butanon gegeben. Man läßt das Reaktionsgemisch auf Raumtemperatur kommen und erhitzt anschließend noch 4 h unter Rückfluß. Nach dem Abkühlen gibt man Ethylacetat zu, wäscht mit Wasser, trennt die organisch Phase ab und trocknet sie mit Magnesiumsulfat. Nach Abziehen des Lösemittels am Rotationsverdampfers bleiben 11,2 g eines Öls [N-(2-Methacryloyloxy-ethyl)-p-toluolsulfonamid] zurück. Das ölige Produkt kann ohne weiteres in die nächste Stufe eingesetzt werden.
c) 11,8 g des in der vorangehenden Stufe erhaltenen rohen N-(2-Methacryloyloxy-ethyl)-p-toluolsulfonamids werden in 50 ml Essigsäure-ethylester gelöst. Unter Rühren gibt man dann bei Raumtemperatur zunächst 10 mg 4-Dimethylamino-pyridin und anschließend 9,2 g Di-tert.-butyl-dicarbonat (Pyrokohlensäure-di-tert.-butylester) zu. Wenn die Umsetzung beendet ist, wäscht man das Reaktionsgemisch mit einer 10 %igen wäßrigen NaOH-Lösung. Dann trennt man die organische Phase ab und trocknet sie mit MgSO₄. Nach dem Abziehen des Lösemittels am Rotationsverdampfer bleiben 14 g eines schwach gefärbten Öls [N-tert.-Butoxycarbonyl-N-(2-methacryloyloxy-ethyl)-p-toluolsulfonamid] zurück.
   ¹H-NMR-Spektrum (60 MHz, CDCl₃ als Lösemittel; chem. Verschiebung in ppm auf der 6-Skala; Anzahl der Protonen in Klammern): 1,3 [9 H], 1,95 [3 H], 2,45 [3 H], 4,0-4,6 [4 H], 5,55 [1 H], 6,15 [1 H], 7,15-7,4 [2 H], 7,65-7,95 [2 H].

### II. N-tert.-Butoxycarbonyl-4-methacryloyloxy-N-phenylbenzolsulfonamid

a) Man löst 200 g 4-Hydroxybenzolsulfonsäure-Na-Salz in 500 ml 2n wäßriger NaOH, verdünnt mit 200 ml Wasser und tropft unter Rühren bei Raumtemperatur 120 g Chlorkohlensäureethylester hinzu. Man läßt über Nacht stehen und saugt den gebildeten Niederschlag (87 g) ab. Abziehen des Lösemittels im Vakuum und Umkristallisation aus Ethanol ergibt weitere 129 g 4-Ethoxycarbonyloxy-benzolsulfonsäure-Na-salz (weißer Feststoff). Die Gesamtausbeute beträgt 216 g (92% der Theorie).
b) 95 g des unter a) erhaltenen, gut getrockneten 4-Ethoxycarbonyloxy-benzolsulfonsäure-Na-salzes werden mit 95 g PCl₅ gut vermischt, wobei sich das Reaktionsgemisch unter Erwärmung verflüssigt. Man erhitzt noch 2 h im Ölbad (Badtemperatur 115°C), läßt über Nacht bei Raumtemperatur stehen, gießt auf Eis und saugt das Sulfochlorid ab. Nach Waschen mit viel kaltem H₂O und Trocknen im Vakuum erhält man 88 g (94 % d. Th.) 4-Ethoxycarbonxyloxy-benzolsulfonylchlorid (weißer Feststoff mit einem Schmelzpunkt (Smp.) von 71-72°C).
c) In 340 ml Anilin werden unter Rühren portionsweise 84 g des unter b) hergestellten 4-Ethoxycarbonyloxy-benzolsulfonylchlorids eingetragen. Man erwärmt 4 h bei 55°C, läßt auf Raumtemperatur abkühlen und gießt die Mischung in 1,7 l halbkonz. Salzsäure ein. Nach Stehen über Nacht dekantiert man die wässr. Phase ab. Zur vollständigen Abspaltung der Schutzgruppe nimmt man den Rückstand in 1,7 l 2n wäßriger NaOH auf, trennt vom Ungelösten ab und fällt das Produkt durch Ansäuern mit HCl aus. Nach Absaugen, Waschen mit H₂O und Trocknen erhält man 54,5 g (70% d. Th.) 4-Hydroxy-benzolsulfanilid (weißer Feststoff, Smp.: 140°C).
d) Man löst 50 g des unter c) hergestellten 4-Hydroxy-benzolsulfanilids in 300 ml Aceton, gibt 35,6 g Methacrylsäureanhydrid hinzu und tropft unter Kühlen und Rühren 22,3 g Triethylamin so hinzu, daß die Temperatur nicht über 10°C steigt. Man läßt auf Raumtemperatur kommen und tropft die Reaktionslösung unter kräftigem Rühren in 3 l H₂O ein. Nach Absaugen und Trocknen erhält man 61 g (96%) 4-Methacryloyloxy-benzolsulfanilid (weißer Feststoff, Smp.: 102-103°C).
e) In 300 ml Aceton werden 50 g des unter d) hergestellten 4-Methacryloyloxy-benzolsulfanilids gelöst, 1 g 4-Dimethylamino-pyridin zugegeben und unter Rühren eine Lösung von 38 g Di-tert-butyldicarbonat in 100 ml Aceton zugetropft. Man rührt 4 h bei Raumtemperatur und fällt in H₂O. Nach Absaugen und Trocknen erhält man 59 g (90%) N-tert.-Butoxycarbonyl-4-methacryloyloxy-N-phenyl-benzolsulfonamid (weißer Feststoff, Smp.: 112-114°C).
   NMR-Spektrum (60 MHz, CDCl₃): 1,3 ppm (9 H); 2,1 (3 H); 5,8 (1 H), 6,4 (1 H); 7,2-7,6 (7 H); 8,0-8,2 (2 H).

### Beispiele 1 bis 20

Die Homopolymerisation von I und II bzw. die Copolymerisation mit verschiedenen Comonomeren, wie Brenzcatechinmonomethacrylat (BMA) oder Styrol erfolgt nach literaturbekannten Methoden. Tabelle 1 enthält eine Auswahl von Polymeren, die unter Verwendung von I und II synthetisiert wurden (Mengenangaben in Molprozenten).

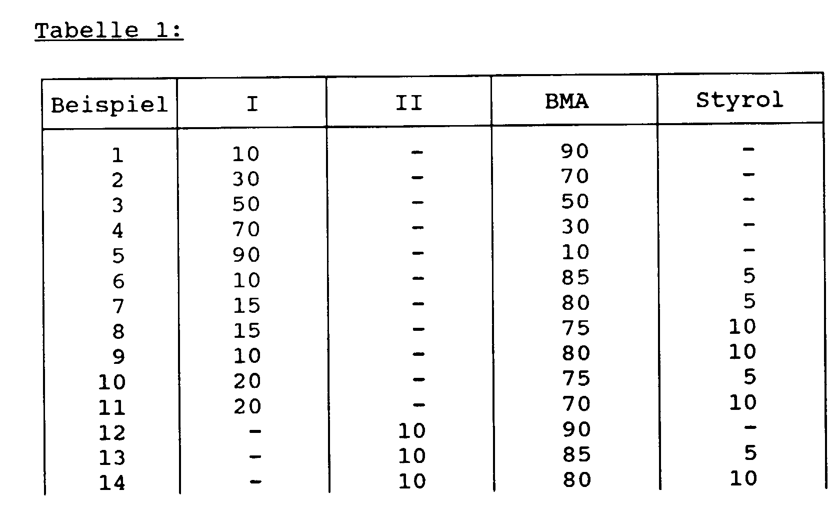

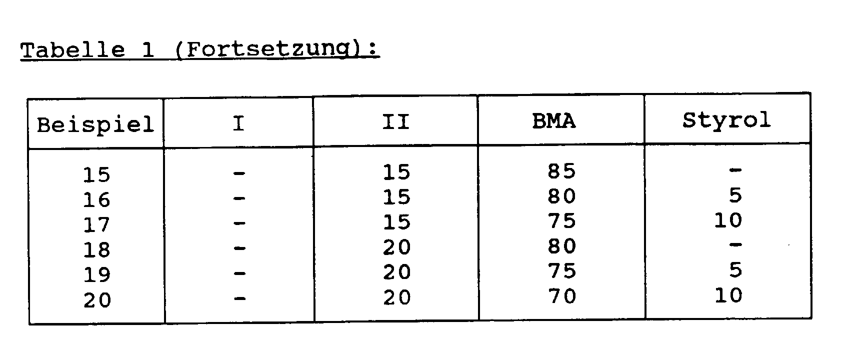

### Beispiel 21 bis 26

Diese Beispiele zeigen die Herstellung und Verarbeitung von erfindungsgemäßen Aufzeichnungsmaterialien.

Eine Platte aus elektrochemisch aufgerauhtem und anodisiertem Aluminium wird mit einer Lösung aus

| | |
|---|---|
| 9,00 Gt | Bindemittel gem. Tab. 1, |
| 0,50 Gt | 4-p-Tolylmercapto-2,5-diethoxy-benzoldiazonium-hexafluoro-phosphat, |
| 0,08 Gt | Kristallviolettbase und |
| 175 Gt | Methylethylketon |

schleuderbeschichtet und bei 100°C im Trockenschrank erhitzt, wobei eine Schichtdicke von 1,9 µm resultiert. Die Platte wird unter einer 5 kW Metallhalogenidlampe im Abstand von 110 cm durch einen Halbtonstufenkeil mit 13 Stufen einer Dichteabstufung von 0,15 belichtet, 1 min bei 100°C nacherwärmt und in einem wäßrig-alkalischen Entwickler der Zusammensetzung

| | |
|---|---|
| 5,5 Gt | Natriummetasilikat·9 H₂O, |
| 3,4 Gt | Trinatriumphosphat·12 H₂O, |
| 0,4 Gt | Mononatriumphosphat wasserfrei und |
| 90,7 Gt | vollentsalztes Wasser |

30 s lang entwickelt. In allen Fällen wird ein positives Abbild der Druckvorlage erhalten. Tabelle 2 gibt an, bei welcher Belichtungszeit die Stufe 4 des Halbtonkeils auf der Platte vollständig offen wiedergegeben wird.

**Tabelle 2:**

| Beispiel | Polymer gemäß Tabelle 1 | Belichtungszeit (s) |
|---|---|---|
| 21 | 8 | 30 |
| 22 | 10 | 30 |
| 23 | 11 | 35 |
| 24 | 18 | 20 |
| 25 | 15 | 18 |
| 26 | 17 | 30 |
| Vergleich* | - | 75 |

| | | |
|---|---|---|
| * Standard-Positivdruckplatte ^{(R)}Ozasol P61 (Hoechst AG) | | |

### Beispiel 27

Dieses Beispiel zeigt die Eignung der erfindungsgemäßen N,N-disubstituierte Sulfonamidgruppierungen enthaltenden Polymere für die Verwendung in positiv arbeitenden Offsetdruckplatten.

Eine Platte aus elektrochemisch aufgerauhtem und anodisiertem Aluminium wird mit einer Lösung aus

| | |
|---|---|
| 9,00 Gt | Terpolymer gem. Tab. 1, Nr. 19, |
| 0,50 Gt | 4-p-Tolylmercapto-2,5-diethoxy-benzoldiazonium-hexafluorophosphat, |
| 0,08 Gt | Kristallviolettbase und |
| 175 Gt | Methylethylketon |

schleuderbeschichtet und bei 100°C im Trockenschrank erhitzt, wobei eine Schichtdicke von 1,9 µm resultiert.

Die Platte wird unter einer 5 kW Metallhalogenidlampe im Abstand von 110 cm 20 s lang durch einen Halbtonstufenkeil mit 13 Stufen einer Dichteabstufung von 0,15 belichtet, 1 min bei 100°C nacherwärmt und in einem wäßrig-alkalischen Entwickler (Zusammensetzung s. Beispiele 21-26) 30 s lang entwickelt. Mit der so erhaltenen Positiv-Druckform werden auf einer Offsetdruckmaschine mehr als 170.000 Drucke in guter Qualität erhalten.

## Patentansprüche

1. Verbindungen der Formeln R¹-SO₂-N(CO-OR²)-R³-O-CO-CR⁴=CH₂ und R¹-N(CO-OR²)-SO₂-R³-O-CO-CR⁴=CH₂, worin
R¹ für einen (C₁-C₂₀)Alkyl-, (C₃-C₁₀) cycloalkyl-, (C₆-C₁₄)Aryl- oder (C₇-C₂₀)Aralkylrest, wobei in den Alkyl enthaltenden Resten einzelne Methylengruppen durch Heteroatome ersetzt sein können,
R² für einen (C₃-C₁₁)Alkyl-, (C₃-C₁₁)Alkenyl- oder (C₇-C₁₁)Aralkulrest,
R³ für einen unsubstituierten oder substituierten (C₁-C₁₂)Alkylen-, (C₃-C₁₂)Cycloalkylen-, (C₆-C₁₅)Arylen- oder (C₈-C₂₀)Arylendialkylrest und
R⁴ für ein Wasserstoffatom oder eine Methylgruppe steht.

2. Verbindungen gemäß Anspruch 1, dadurch gekennzeichnet, daß R² für einen (C₃-C₆)Alkylrest, bevorzugt für einen Isopropyl-, sec.-Butyl- oder tert.-Butylrest steht.

3. Polymere mit mindestens 5 mol-% an Einheiten mit Seitengruppen der Formel(n) -R³-N(CO-OR²)-SO₂-R¹ (I) und/oder -R³-SO₂-N(CO-OR²)-R¹ (II), worin
R¹ ein (C₁-C₂₀)Alkyl-, (C₃-C₁₀)Cycloalkyl-, (C₆-C₁₄)Aryl- oder (C₇-C₂₀)Aralkylrest, wobei in den Alkyl enthaltenden Resten einzelne Methylengruppen durch Heteroatome ersetzt sein können,
R² ein (C₃-C₁₁)Alkyl-, (C₃-C₁₁)Alkenyl- oder (C₇-C₁₁)Aralkylrest, und
R³ ein unsubstituierter oder substituierter (C₁-C₁₂)Alkylen-, (C₃-C₁₂)Cycloalkylen-, (C₆-C₁₅)Arylen- oder (C₈-C₂₀)Arylendialkylrest ist.

4. Polymere gemäß Anspruch 3, dadurch gekennzeichnet, daß der Rest R² für einen Isopropyl-, sec.-Butyl- oder tert.-Butylrest steht.

5. Strahlungsempfindliches Gemisch mit
a) einer Verbindung, die unter der Einwirkung aktinischer Strahlung Säure bildet, und
b) einer säurespaltbaren Verbindung, deren Spaltprodukte in einem wäßrig-alkalischen Entwickler eine höhere Löslichkeit zeigen als die Ausgangsverbindung,
dadurch gekennzeichnet, daß die säurespaltbare Verbindung b) ein Polymer gemäß Anspruch 3 oder 4 ist.

6. Strahlungsempfindliches Gemisch gemäß Anspruch 5, dadurch gekennzeichnet, daß der Anteil der säurebildenden Verbindung(en) a) etwa 0,1 bis 20 Gew.-%, bevorzugt 0,2 bis 10% Gew.-%, jeweils bezogen auf das Gesamtgewicht der Feststoffe in dem Gemisch, beträgt.

7. Strahlungsempfindliches Gemisch gemäß Anspruch 5, dadurch gekennzeichnet, daß der Anteil der säurespaltbaren Verbindung(en) b) 30 bis 98 Gew.-%, bevorzugt 50 bis 95 Gew.-%, jeweils bezogen auf das Gesamtgewicht der Feststoffe in dem Gemisch beträgt.

8. Strahlungsempfindliches Gemisch gemäß Anspruch 5, dadurch gekennzeichnet, daß es zusätzlich ein organisches, polymeres Bindemittel enthält.

9. Strahlungsempfindliches Gemisch gemäß Anspruch 8, dadurch gekennzeichnet, daß der Anteil des polymeren Bindemittels 5 bis 70 Gew.-%, bevorzugt 20 bis 50 Gew.-%, jeweils bezogen auf das Gesamtgewicht der Feststoffe in dem Gemisch, beträgt.

10. Aufzeichnungsmaterial mit einem Träger und einer strahlungsempfindlichen Schicht, dadurch gekennzeichnet, daß die Schicht aus dem strahlungsempfindlichen Gemisch gemäß einem oder mehreren der Ansprüche 5 bis 9 besteht.

## Claims

1. Compounds of the formulae R¹-SO₂-N(CO-OR₂)-R³-O-CO-CR⁴=CH₂ or R¹-N(CO-OR²)-SO₂-R³-O-CO-CR⁴=CH₂, in which
R¹ is a (C₁-C₂₀)alkyl, (C₃-C₁₀)cydoalkyl, (C₆-C₁₄)aryl or (C₇-C₂₀)aralkyl radical, individual methylene groups in the radicals containing alkyl being optionally replaced by heteroatoms,
R² is a (C₃-C₁₁)alkyl, (C₃-C₁₁)alkenyl or (C₇-C₁₁)aralkyl radical,
R³ is an unsubstituted or substituted (C₁-C₁₂)alkylene, (C₃-C₁₂)cycloalkylene, (C₆-C₁₅)arylene or (C₈-C₂₀)arylenedialkyl radical and
R⁴ is a hydrogen atom or a methyl group.

2. Compounds as claimed in claim 1, wherein R² is a (C₃-C₆)alkyl radical, preferably an isopropyl, sec-butyl or tert-butyl radical.

3. Polymers having at least 5 mol % of units with lateral groups of the formula(e) -R³-N(CO-OR²)-SO₂-R¹ (I) and/or -R³-SO₂-N(CO-OR²)-R¹ (II), in which
R¹ is a (C₁-C₂₀)alkyl, (C₃-C₁₀)cycloalkyl, (C₆-C₁₄)aryl or (C₇-C₂₀)aralkyl radical, individual methylene groups in the radicals containing alkyl being optionally replaced by heteroatoms,
R² is a (C₃-C₁₁)alkyl;(C₃-C₁₁)alkenyl or (C₇-C₁₁)aralkyl radical, and
R³ is an unsubstituted or substituted (C₁-C₁₂)alkylene, (C₃-C₁₂)cycloalkylene, (C₆-C₁₅)arylene or (C₈-C₂₀)arylenedialkyl radical.

4. Polymers as claimed in claim 3, wherein the radical R² is an isopropyl, sec-butyl or tert-butyl radical.

5. Radiation sensitive mixture comprising
a) a compound which under the influence of actinic radiation forms acid, and
b) an acid-cleavable compound whose cleavage products in an aqueous-alkaline developer have a higher solubility than the starting compound,
wherein the acid-cleavable compound b) is a polymer according to claim 3 or 4.

6. The radiation-sensitive mixture as claimed in claim 5, wherein the proportion of the acid-forming compound(s) a) is from approximately 0.1 to 20% by weight, preferably from 0.2 to 10% by weight, in each case based on the total weight of the solids in the mixture.

7. The radiation-sensitive mixture as claimed in claim 5, wherein the proportion of the acid-cleavable compound(s) b) is from approximately 30 to 98% by weight, preferably from 50 to 95% by weight, in each case based on the total weight of the solids in the mixture.

8. The radiation-sensitive mixture as claimed in claim 5, which comprises, in addition, an organic polymer binder.

9. The radiation-sensitive mixture as claimed in claim 8, wherein the proportion of the polymeric binder is from approximately 5 to 70% by weight, preferably from 20 to 50% by weight, in each case based on the total weight of the solids in the mixture.

10. A recording material comprising a support and a radiation-sensitive layer, wherein the layer comprises the radiation-sensitive mixture according to one or more of claims 5 to 9.

## Revendications

1. Composés de formules R¹-SO₂-N(CO-OR²)-R³-O-CO-CR⁴=CH₂ et R¹-N(CO-OR²)-SO₂-R³-O-CO-CR⁴=CH₂, dans lesquelles
R¹ est un radical alkyle en C₁-C₂₀, cycloalkyle en C₃-C₁₀, aryle en C₆-C₁₄ ou aralkyle en C₇-C₂₀, dans lequel des groupes méthylènes particuliers dans les groupes contenant des radicaux alkyles peuvent être remplacés par des hétéroatomes, et
R² est un radical alkyle en C₃-C₁₁, alkényle en C₃-C₁₁ ou aralkyle en C₇-C₁₁,
R³ est un radical substitué ou non substitué alkylène en C₁-C₁₂, cycloalkylène en C₃-C₁₂, arylène en C₆-C₁₅ ou arylènedialkyle en C₈-C₂₀,
R⁴ est un atome d'hydrogène ou un radical méthyle.

2. Composés selon la revendication 1, caractérisés en ce que, R² est un radical alkyle en C₃-C₆, de préférence un radical isopropyle, sec.-butyle ou tert.-butyle.

3. Polymères avec au moins 5% en moles de motifs contenant des groupes latéraux formule(s) -R³-N(CO-OR²)-SO₂-R¹ (I) et/ou -R³-SO₂-N(CO-OR²)-R¹ (II), dans lesquelles
R¹ est un radical alkyle en C₁-C₂₀, cycloalkyle en C₃-C₁₀, aryle en C₆-C₁₄ ou aralkyle en C₇-C₂₀, dans lequel des groupes méthylènes particuliers dans les groupes contenant des radicaux alkyles peuvent être remplacés par des hétéroatomes,
R² est un radical alkyle en C₃-C₁₁, alkényle en C₃-C₁₁ ou aralkyle en C₇-C₁₁, et
R³ est un radical substitué ou non substitué alkylène en C₁-C₁₂, cycloalkylène en C₃-C₁₂, arylène en C₆-C₁₅ ou arylènedialkyle en C₈-C₂₀,

4. Polymères selon la revendication 3, caractérisés en ce que, le radical R² représente un radical isopropyle, sec.-butyle ou tert.-butyle.

5. Composition sensible aux rayonnements contenant
a) un composé formant un acide sous l'action de rayonnement actinique, et
b) un composé décomposable par un acide dont les produits de décomposition présentent dans un révélateur de type alcalin en solution aqueuse une plus forte solubilité que le composé initial,
caractérisée en ce que, le composé décomposable par un acide b) est un polymère selon la revendication 3 ou 4.

6. Composition sensible aux rayonnements selon la revendication 5, caractérisée en ce que, la teneur en composé(s) a) formant un acide est d'environ 0,1 à 20% en poids, de préférence 0,2 à 10% en poids, par rapport au poids total des matières solides dans la préparation.

7. Composition sensible aux rayonnements selon la revendication 5, caractérisée en ce que, la teneur en composé(s) b) décomposable par un acide est d'environ 30 à 98% en poids, de préférence 50 à 95% en poids par rapport au poids total des matières solides dans la préparation.

8. Composition sensible aux rayonnements selon la revendication 5, caractérisée en ce qu'elle contient de plus un liant organique polymère.

9. Composition sensible aux rayonnements selon la revendication 8, caractérisée en ce que, la teneur en liant polymère est de 5 à 70% en poids, de préférence 20 à 50% en poids, par rapport au poids total des matières solides dans le mélange.

10. Matériau d'enregistrement avec un support et une couche sensible aux rayonnements, caractérisé en ce que, la couche est constituée d'une composition sensible aux rayonnements selon une ou plusieurs des revendications 5 à 9.
